# EUROPÄISCHE PATENTSCHRIFT

(11) **EP 1 906 939 B1**
(45) Veröffentlichungstag und Bekanntmachung des Hinweises auf die Patenterteilung: **01.06.2016**
(21) Anmeldenummer: 06724062.2
(22) Anmeldetag: 05.04.2006
(51) Int. Cl.: A61K 9/50

(54) **VERWENDUNG EINES TEILNEUTRALISIERTEN, ANIONISCHEN (METH)ACRYLAT- COPOLYMERS ALS ÜBERZUG FÜR DIE HERSTELLUNG EINER ARZNEIFORM MIT EINER WIRKSTOFFFREISETZUNG BEI ERNIEDRIGTEN PH-WERTEN**
USE OF A PARTIALLY NEUTRALIZED, ANIONIC (METH)ACRYLATE COPOLYMER AS A COATING FOR THE PRODUCTION OF A MEDICAMENT RELEASING ACTIVE SUBSTANCE AT REDUCED PH VALUES
UTILISATION D'UN COPOLYMERE (METH)ACRYLIQUE ANIONIQUE PARTIELLEMENT NEUTRALISE COMME ENROBAGE DANS LA PRODUCTION D'UNE FORME MEDICAMENTEUSE LIBERANT LE PRINCIPE ACTIF A UN PH REDUIT

(30) Priorität: 12.07.2005 DE 102005032806
(43) Veröffentlichungstag der Anmeldung: 09.04.2008
(73) Patentinhaber: Evonik Röhm GmbH, 64293 Darmstadt (DE)
(72) Erfinder: PETEREIT, Hans-Ulrich, 64291 Darmstadt (DE); ASSMUS, Manfred, 64404 Bickenbach (DE)
(86) Internationale Anmeldenummer: PCT/EP2006/003115
(87) Internationale Veröffentlichungsnummer: WO 2007/006353

(56) Entgegenhaltungen:
- EP-A2- 0 393 747
- EP-A2- 0 636 366
- WO-A-00/12064
- WO-A-00/33821
- WO-A-03/072087
- WO-A-2006/087027
- WO-A2-02/096394
- WO-A2-2005/032513

## Beschreibung

### Gebiet der Erfindung

Die Erfindung betrifft die Verwendung eines teilneutralisierten, anionischen (Meth)acrylat-Copolymers als Überzug für die Herstellung einer Arzneiform mit einer Wirkstofffreisetzung bei erniedrigten pH-Werten.

### Stand der Technik

EP 0 088 951 A2 beschreibt ein Verfahren zum Überziehen von Arzneiformen mittels eines in Wasser dispergierten Überzugsmittels. Für die Redispergierung von Carboxylgruppen-haltigen (Meth)acrylatcopolymeren von Pulvern zu Dispersionen wird die Teilneutralisation der Carboxylgruppen empfohlen. Die Salzbildung der sauren Gruppen tritt durch Umsetzung mit einer Base ein. Als Basen kommen Alkalien, wie z.B. Natronlauge, Kalilauge, Soda, Pottasche, Natriumbikarbonat, Trinatriumphosphat, Trinatriumcitrat oder Ammoniak oder physiologisch verträgliche Amine, wie Triethanolamin oder Tris-(hydroxymethyl)-aminomethan, in Betracht. Günstig in Bezug auf die Redispergierung ist ein Neutralisationsgrad von 0,1 bis 10 Gew.-% der im Copolymeren enthaltenen Carboxylgruppen.

WO 2003/072087 beschreibt eine Arzneiform und ein Verfahren zu ihrer Herstellung. Die Arzneiform ist mit einem anionischen (Meth)acrylatcopolymeren überzogen, das bei Bedarf teilneutralisiert werden kann. Um eine Lösung des anionischen Copolymers herzustellen ist in der Regel eine teilweise oder vollständige Neutralisation der Säuregruppen nötig. Das anionische Copolymer kann z. B. nach und nach in einer Endkonzentration von 1 bis 40 Gew.-% in Wasser eingerührt werden und dabei teilweise oder vollständig neutralisiert werden durch Zugabe einer basischen Substanz wie z. B. NaOH, KOH, Ammoniumhydroxyd oder organische Basen wie z. B. Triethanolamin. Es ist auch möglich ein Pulver des Copolymeren einzusetzen, dem bereits bei seiner Herstellung zum Zweck der (Teil)neutralisation eine Base z. B. NaOH zugesetzt wurde, so daß das Pulver ein bereits (teil)neutralisiertes Polymer ist. Der pH-Wert der Lösung liegt in der Regel über 4, z. B. im Bereich von 4 bis ca. 7.

WO 2004/096185 beschreibt ebenfalls eine Arzneiform und ein Verfahren zu ihrer Herstellung. Die Arzneiform ist mit einem von dem der WO 2003/072087 verschiedenen anionischen (Meth)acrylatcopolymeren überzogen, das bei Bedarf teilneutralisiert werden kann. Um eine Lösung des anionischen Copolymers herzustellen ist in der Regel eine teilweise oder vollständige Neutralisation der Säuregruppen nötig. Das anionische Copolymer kann z. B. nach und nach in einer Endkonzentration von 1 bis 40 Gew.-% in Wasser eingerührt werden und dabei teilweise oder vollständig neutralisiert werden durch Zugabe einer basischen Substanz wie z. B. NaOH, KOH, Ammoniumhydroxyd oder organische Basen wie z. B. Triethanolamin. Es ist auch möglich ein Pulver des Copolymeren einzusetzen, dem bereits bei seiner Herstellung zum Zweck der (Teil)neutralisation eine Base z. B. NaOH zugesetzt wurde, so daß das Pulver ein bereits (teil)neutralisiertes Polymer ist. Der pH-Wert der Lösung liegt in der Regel über 4, z. B. im Bereich von 4 bis ca. 7.

### Aufgabe und Lösung

Anionische (Meth)acrylatcopolymere, z. B. vom Typ EUDRAGIT^{®} L, EUDRAGIT® L100-55, EUDRAGIT® S oder EUDRAGIT® FS, sind als darmsaftlösliche Überzüge für Arzneiformen bekannt. In Abhängigkeit von der Monomerzusammensetzung, insbesondere aber in Abhängigkeit von Gehalt anionischer Gruppen, sind die anionische (Meth)acrylatcopolymere durch spezifische Auflösungs-pH-Werte in Darmsaft oder in künstlichem Darmsaft charakterisiert. Je nach Polymer-Typ liegen die spezifischen Auflösungs-pH-Werte bzw. die pH-Werte des spezifischen Beginns der Auflösung im Bereich von z. B. pH 5,5 bis 7,5. Ab dem für das jeweilige anionische (Meth)acrylatcopolymere spezifischen Auflösungs-pH-Wert und darüber setzen damit überzogene Arzneiformen den enthaltenen Wirkstoff frei. Die spezifischen Auflösungs-pH-Werte charakterisieren somit den Beginn der Wirkstofffreisetzung.

Allerdings kann es im Übergangsbereich vom Magen in das Duodenums durch den Einfluss der des Speisebreis und der darin enthaltenen Magensäure in nicht vorhersehbarer zu pH-Verschiebungen kommen, die sich bis ins Jejunum fortsetzen. Es kann daher vorkommen, dass ein Wirkstoff der theoretisch bereits unmittelbar im Duodenum bei einem pH-Wert von 5,5 oder 6,0 freigesetzt werden soll, in-vivo noch nicht freigesetzt wird, weil nahrungsaufnahmebedingt in der jeweiligen Situation der pH-Wert noch unterhalb des spezifischen Auflöse-pH-Werts des Polymerüberzugs liegt.

Für eine schnelle und vollständige Wirkung von Arzneistoffen ist, wegen seines Resorptionsvermögens, besonders das sich an den Magen anschließende Duodenum von Interesse. In diesem Bereich des Dünndarms, der durch starke Motilität gekennzeichnet ist, schwanken die pH-Werten zwischen 2 und 6 zu rechnen, abhängig von Inhalt und Motilitätscyclus des Magens. Die Passagezeiten durch das Duodenum sind kurz und liegen im Bereich von 15 bis 30 min.

Für eine Vielzahl von Wirkstoffen, z. B. Schmerzmitteln, wird ein rasches Einsetzen der Wirkung, gleichbedeutend mit früher Resorption im Duodenum, angestrebt. Gleichzeitig gibt es eine Vielzahl von Wirkstoffen, die ausreichend löslich und (säure-)stabil sind und somit theoretisch für eine Freisetzung im sauren Milieu geeignet sind. Es wäre daher günstig, wenn man den spezifischen Auflösungs-pH-Wert von anionischen (Meth)acrylatcopolymeren in den sauren Bereich vorverlegen könnte, um diese zum Überziehen von Arzneiformen zu verwenden, die Wirkstoffe mit ausreichender Säurestabilität enthalten und bei denen eine rasche Resorption erwünscht ist.

Es ist bekannt anionische (Meth)acrylatcopolymere in teilneutralisierter Form einzusetzen. Dadurch wird eine verbesserte Löslichkeit des Polymeren in Wasser und eine Stabilisierung der Polymerdispersionen erreicht. Als Basen für die Teilneutralisation werden in der Regel Substanzen wie NaOH, KOH, Ammoniumhydroxyd oder organische Basen, wie z. B. Triethanolamin, empfohlen. Die Verwendung von teilneutralisierten, anionischen (Meth)acrylat-Copolymeren zum gezielten Absenken der spezifischen Auflösungs-pH-Werte von anionischen (Meth)acrylatcopolymeren in den sauren Bereich ist nicht bekannt.

Es wurde daher als Aufgabe gesehen, eine, mit einem anionischen (Meth)acrylatcopolymeren überzogene Arzneiform bereitzustellen, die in-vitro und in-vivo einen nicht unerheblichen Anteil des enthaltenen Wirkstoffs bereits vor dem Erreichen spezifischen Auflösungs-pH-Wert des anionischen (Meth)acrylatcopolymeren in verhältnismäßig kurzer Zeit freizugeben vermag.

### Die Aufgabe wird gelöst durch die

Verwendung eines teilneutralisierten, anionischen (Meth)acrylat-Copolymers, bestehend aus radikalisch polymerisierten Einheiten von 40 bis 60 Gew.-% C₁-bis C₄-Alkylestern der Acryl- oder der Methacrylsäure und 60 bis 40 Gew.-% (Meth)acrylat-Monomeren mit einer anionischen Gruppe, wobei mehr als 10 bis 50 % der enthaltenen anionischen Gruppen mittels einer Base neutralisiert sind,

zur Herstellung einer Arzneiform mit wirkstoffhaltigem Kern, die mit dem teilneutralisierten, anionischen (Meth)acrylat-Copolymer überzogen ist und mindestens 30 % des enthaltenen Wirkstoffs in 30 Minuten bei einem pH-Wert freisetzt, bei dem der Wirkstoff ausreichend löslich und stabil ist und bei dem eine entsprechende Arzneiform, die mit dem nicht neutralisierten anionischen (Meth)acrylat-Copolymer überzogen ist, weniger als 10 % des enthaltenen Wirkstoffs in 30 Minuten freisetzen würde,
wobei der Überzug der Arzneiform bis zu 30 Gew.-% weiterer filmbildender Polymere, die keine oder nur in unwesentliche Mengen ionische Seitengruppen aufweisen, enthalten kann.

### Ausführung der Erfindung

Die Erfindung betrifft die Verwendung eines teilneutralisierten, anionischen (Meth)acrylat-Copolymers, bestehend aus radikalisch polymerisierten Einheiten von 40 bis 60 Gew.-% C₁-bis C₄-Alkylestern der Acryl- oder der Methacrylsäure und 40 bis 60 Gew.-% (Meth)acrylat-Monomeren mit einer anionischen Gruppe, wobei mehr als 10 bis 50 % oder bevorzugt mehr als 10 bis 20 % oder bevorzugt mehr als 20 bis 40 %, der enthaltenen anionischen Gruppen mittels einer Base neutralisiert sind,
zur Herstellung einer Arzneiform mit wirkstoffhaltigem Kern, die mit dem teilneutralisierten, anionischen (Meth)acrylat-Copolymer überzogen ist und mindestens 30, bevorzugt mindestens 50, insbesondere mindestens 70 % des enthaltenen Wirkstoffs in 30 Minuten bei einem pH-Wert freisetzt bei dem der Wirkstoff ausreichend löslich und stabil ist und bei dem eine entsprechende Arzneiform, die mit dem nicht neutralisierten, anionischen (Meth)acrylat-Copolymer überzogen ist, weniger als 10, insbesondere weniger als 8, bevorzugt weniger als 5 % des enthaltenen Wirkstoffs in 30 Minuten freisetzt bzw. freisetzen würde. Die Wirkstofffreisetzung kann beim jeweiligen pH-Wert analog nach USP 28 ermittelt werden (siehe auch die Beispiele). Durch die Neutralisation von bevorzugt mehr als 10 bis 20 % oder bevorzugt mehr als 20 bis 40 % der enthaltenen anionischen Gruppen kann der spezifische Auflösungs-pH-Wert des (Meth)acrylatcopolymeren, je nach Neutralisationgrad, um 0,5 bis 1,5, bevorzugt mehr als 1,5 bis 2,5 bzw. um mehr als 2,5 bis 3,5 pH-Einheiten gesenkt bzw. erniedrigt werden. Je nach Wirkstoff kann die geeignete Variante gewählt werden, um eine Wirkstofffreisetzung beim entsprechend erniedrigten pH-Wert zu gewährleisten.

### Anionisches (Meth)acrylat-Copolymer

Das anionische (Meth)acrylat-Copolymere besteht zu 60 bis 40 Gew.-% aus radikalisch polymerisierten C₁- bis C₄-Alkylestern der Acryl- oder der Methacrylsäure und zu 40 bis 60 Gew.-% aus (Meth)acrylat-Monomeren mit einer anionischen Gruppe.

In der Regel addieren sich die genannten Anteile zu 100 Gew.-%. Es können jedoch zusätzlich, ohne daß dies zu einer Beeinträchtigung oder Veränderung der wesentlichen Eigenschaften führt, geringe Mengen im Bereich von 0 bis 10, z. B. 1 bis 5 Gew.-% weiterer vinylisch copolymerisierbarer Monomere, wie z. B. Hydroxyethylmethacrylat oder Hydroxyethylacrylat enthalten sein. Bevorzugt sind keine weiteren vinylisch copolymerisierbaren Monomere enthalten.

C₁- bis C₄-Alkylestern der Acryl- oder Methacrylsäure sind insbesondere Methylmethacrylat, Ethylmethacrylat, Butylmethacrylat, Methylacrylat, Ethylacrylat und Butylacrylat.

Ein (Meth)acrylat-Monomer mit einer anionischen Gruppe ist z. B. Acrylsäure, bevorzugt ist Methacrylsäure.

Geeignet für die Zwecke der Erfindung sind insbesondere anionische (Meth)acrylat Copolymere aus 40 bis 60, Gew.-% Methacrylsäure und 60 bis 40 Gew.-% Methylmethacrylat oder 60 bis 40 Gew.-% Ethylacrylat (Typen EUDRAGIT® L oder EUDRAGIT® L100-55). Durch Teilneutralisation von mindestens 4 %, bevorzugt 4 bis 40 % oder bevorzugt 4 bis 10 oder bevorzugt mehr als 10 bis 20 % oder bevorzugt mehr als 20 bis 40 % der enthaltenen anionischen Gruppen kann, je nach Neutralisationgrad, der spezifische Auflösungs-pH-Wert um 0,5 bis 3,5 pH-Einheiten gesenkt werden. EUDRAGIT® L ist ein Copolymer aus 50 Gew.-% Methylmethacrylat und 50 Gew.-% Methacrylsäure. Der pH-Wert des Beginns der spezifischen Wirkstofffreisetzung in Darmsaft oder künstlichem Darmsaft (spezifischer Auflösungs-pH-Wert) kann mit pH 6,0 angegeben werden. Durch Teilneutralisation von mindestens 4 %, bevorzugt 4 bis 40 % oder bevorzugt 4 bis 10 oder bevorzugt mehr als 10 bis 20 % oder bevorzugt mehr als 20 bis 40 % der enthaltenen anionischen Gruppen kann, je nach Neutralisationgrad, der spezifische Auflösungs-pH-Wert um 0,5 bis 3,5 pH-Einheiten gesenkt werden. EUDRAGIT® L100-55 ist ein Copolymer aus 50 Gew.-% Ethylacrylat und 50 Gew.-% Methacrylsäure. EUDRAGIT® L 30D-55 ist eine Dispersion, enthaltend 30 Gew.-% EUDRAGIT® L100-55. Der pH-Wert des Beginns der spezifischen Wirkstofffreisetzung in Darmsaft oder künstlichem Darmsaft (spezifischer Auflösungs-pH-Wert) kann mit pH 5,5 angegeben werden. Durch Teilneutralisation von mindestens 4 %, bevorzugt 4 bis 40 % oder bevorzugt 4 bis 10 oder bevorzugt mehr als 10 bis 20 % oder bevorzugt mehr als 20 bis 40 % der enthaltenen anionischen Gruppen kann, je nach Neutralisationgrad, der spezifische Auflösungs-pH-Wert um 0,5 bis 3,5 pH-Einheiten gesenkt werden.

### Herstellung der anionischen (Meth)acrylatcopolymere

Die Herstellung der anionischen (Meth)acrylatcopolymere kann in an sich bekannter Weise durch radikalische Polymerisation der Monomeren erfolgen (siehe z. B. EP 0 704 207 A2, EP 0 704 208 A2, WO 2003/072087, WO 2004/096185). Die Copolymere sind in an sich bekannter Weise durch radikalische Emulsionspolymerisation in wäßriger Phase in Gegenwart von vorzugsweise anionischen Emulgatoren herstellbar, beispielsweise nach dem in DE-C 2 135 073 beschriebenen Verfahren.

Das Copolymerisat kann nach gängigen Verfahren der radikalischen Polymerisation kontinuierlich oder diskontinuierlich (Batch-Verfahren) in Gegenwart radikalbildender Initiatoren und gegebenenfalls Reglern zur Einstellung des Molekulargewicht in Substanz, in Lösung, durch Perlpolymerisation oder in Emulsion hergestellt werden. Das mittlere Molekulargewicht Mw (Gewichtsmittel, bestimmt z. B. durch Messung der Lösungsviskosität) kann z. B. im Bereich von 80.000 bis 1.000.000 (g/mol) liegen. Bevorzugt ist die Emulsionspolymerisation in wäßriger Phase in Gegenwart wasserlöslicher Initiatoren und (vorzugsweise anionischer) Emulgatoren.
Im Falle der Substanzpolymerisation kann das Copolymer in fester Form durch Brechen, Extrusion, Granulieren oder Heißabschlag erhalten werden.

Die (Meth)acrylatcopolymere werden in an sich bekannter Weise durch radikalische Substanz-, Lösungs-, Perl- oder Emulsionspolymerisation erhalten. Sie müssen vor der Verarbeitung durch geeignete Mahl-, Trocken- oder Sprühprozesse in den erfindungsgemäßen Teilchengrößenbereich gebracht werden. Dies kann durch einfaches Brechen extrudierter und abgekühlter Granulatstränge oder Heißabschlag erfolgen.

Insbesondere bei Mischung mit weiteren Pulvern oder Flüssigkeiten kann der Einsatz von Pulvern vorteilhaft sein. Geeignete Gerätschaften zur Herstellung der Pulver sind dem Fachmann geläufig, z. B. Luftstrahlmühlen, Stiftmühlen, Fächermühlen. Gegebenenfalls können entsprechende Siebungsschritte einbezogen werden. Eine geeignete Mühle für industrielle Großmengen ist zum Beispiel eine Gegenstrahlmühle (Multi Nr. 4200), die mit ca. 6 bar Überdruck betrieben wird.

### Teilneutralisation

Die Erfindung betrifft die Verwendung eines teilneutralisierten anionischen (Meth)acrylat-Copolymers, bestehend aus radikalisch polymerisierten Einheiten von 40 bis 60 Gew.-% C₁- bis C₄-Alkylestern der Acryl- oder der Methacrylsäure und 40 bis 60 Gew.-% (Meth)acrylat-Monomeren mit einer anionischen Gruppe, wobei mehr als 10 bis 50 %, besonders bevorzugt 12 bis 40, insbesondere mehr als 15 bis 35 % der enthaltenen anionischen Gruppen mittels einer Base neutralisiert sind.

Geeignet für die erfindungsgemäße Teilneutralisation des (Meth)acrylatcopolymeren sind Basen, insbesondere anorganische Basen, bevorzugt solche mit einem Molekulargewicht von höchstens 150. Insbesondere sind geeignet sind z. B. Natronlauge (NaOH), Kalilauge (KOH) Ammoniumhydroxyd oder organische Basen wie z. B. Triethanolamin, Soda, Pottasche, Natriumbikarbonat, Trinatriumphosphat, Trinatriumcitrat oder Ammoniak oder physiologisch verträgliche Amine, wie Triethanolamin oder Tris-(hydroxymethyl)-aminomethan. Besonders bevorzugt ist NaOH.

Das Molekulargewicht der genannten Substanzen ist bekannt bzw. kann anhand der in Molekül vorhandenen Atome anhand der Atomgewichte errechnet werden.

Nicht geeignet sind bzw. von der Erfindung ausdrücklich ausgenommen sind kationische, organische Basen mit einem M_{w} größer 150, weil diese das Wirkstofffreigabeverhalten in anderer, nicht erfindungsgemäßer Weise beeinflussen. Auszuschließen sind u.a. natürliche oder synthetische Oligomere oder Polymere, z. B. aus 3 bis 100, bevorzugt 5 bis 25 Einheiten, von Histidin, Arginin oder Lysin, Poly-Histidine, Poly-Arginine, Poly-Lysine, kationische bzw. zwitterionische Phopholipide, wie z. B. Phosphatidylcholin, Ribonukleoside: Kondensationsprodukte der Hydroxylfunktion am Kohlenstoffatom 1 der Ribose mit der heterocyklischen Aminofunktion der Basen Adenin, Guanin, Cytosin, Thymin oder Uracil, entsprechend dem Vorkommen in der RNA; Desoxyribonukleoside: Kondensationsprodukte der Hydroxylfunktion am Kohlenstoffatom 1 der Desoxyribose mit der heterocyklischen Aminofunktion der Basen Adenin, Guanin, Cytosin, Thymin oder Uracil, entsprechend dem Vorkommen in der DNA; Basen aus kationischen oberflächenaktiven Hilfsstoffen oder Emulgatoren, wie Benzalkonium (CAS RN: 8001-54-5), Benzethonium (CAS 121-54-0), Cetalkonium (CAS 122-18-9), Cetrimide (CAS 8044-71-1), Cetrimonium (CAS 57-09-0), Cetylpyridinium (CAS 123-03-5), Stearalkonium (CAS 122-19-0), Diallyldimethylammonium (CAS 230-993-8)

### Dispersionen

Das teilneutralisierte (Meth)acrylat-Copolymer kann z. B. in Form einer wäßrigen Dispersion mit 10 bis 50-prozentigen Feststoffanteil vorliegen.

Das teilneutralisierte (Meth)acrylat-Copolymer kann in Form eines redispergierbaren Pulvers vorliegen, welches aus einer Dispersion z. B. durch Sprühtrocknung gewonnen wurde.

### Dispersionen / Teilneutralisation

Das Emulsionspolymerisat wird vorzugsweise in Form einer 10- bis 50-gew.-prozentigen, insbesondere 20 bis 40-prozentigen wäßrigen Dispersion erzeugt und angewendet. Als Handelsform ist ein Feststoffgehalt von 30 Gew.% bevorzugt. Der Gewichtsmittelwert Latex-Teilchengröße (Radius) beträgt in der Regel 40 bis 100 nm, vorzugsweise 50 bis 70 nm, was eine verarbeitungstechnisch günstige Viskosität unter 1000 mPa·s gewährleistet. Die Teilchengröße kann durch Laserbeugung, z. B. mit dem Mastersizer 2000 (Fa. Malvern), bestimmt werden.

Das anionische Copolymer kann z. B. nach und nach in einer Endkonzentration von 1 bis 40 Gew.-% in Wasser eingerührt werden und dabei wie beschrieben teilweise werden durch Zugabe einer erfindungsgemäßen basischen Substanz wie z. B. NaOH. Es ist auch möglich ein Pulver des Copolymeren einzusetzen, dem bereits bei seiner Herstellung zum Zweck der (Teil)neutralisation eine Base z. B. NaOH zugesetzt wurde, so daß das Pulver ein bereits (teil)neutralisiertes Polymer ist. Der pH-Wert der Lösung liegt in der Regel über 4, z. B. im Bereich von 4 bis ca. 6. Man kann dabei auch z. B. Mischungen von Chargen von voll- oder teilneutralisierten Dispersionen mit nicht neutralisierten Dispersionen oder Mischungen von Chargen mit unterschiedlichem Teilneutralisationsgrad vornehmen und in beschriebener Weise weiterverarbeiten, d. h. die Mischung für Überzüge verwenden oder zunächst zu einem Pulver gefrier- oder sprühtrocknen. Möglich ist ebenfalls die Mischung eines Pulvers aus nicht neutralisiertem Polymer mit einer entsprechenden Menge an fester Base, so dass die Neutralisation erst bei der Redispergierung in Wasser entsteht.

Die Dispersion kann z. B. auch in an sich bekannter Weise sprühgetrocknet oder gefriergetrocknet werden und im Form eines redispergierbaren Pulvers bereitgestellt werden (siehe z. B. EP-A 0 262 326). Alternative Verfahren sind die Gefriertrocknung oder Coagulation uns Abquetschen des Wassers in einem Extruder mit anschließender Granulation (siehe z. B. EP-A 0 683 028).

Copolymer-Dispersionen aus sprüh- oder gefriergetrockneten und redispergierten Pulvern können eine erhöhte Scherstabilität aufweisen. Dies ist insbesondere beim Sprühauftrag von Vorteil. Durch Zusatz von Emulgatoren kann die Scherstabilität erhöht werden. Bevorzugt ist ein anionischer Emulgator in Menge von 0,1 bis 2 Gew.-% enthalten. Besonders bevorzugt ist Natiumlaurylsulfat als Emulgator.

### Verwendung des teilneutralisierten (Meth)acrylatcopolymeren

Das teilneutralisierte, anionische (Meth)acrylat-Copolymere kann erfindungsgemäß zur Herstellung einer Arzneiform mit wirkstoffhaltigem Kern verwendet werden, die mit dem teilneutralisierten anionischen (Meth)acrylat-Copolymer überzogen ist und mindestens 30 % des enthaltenen Wirkstoffs in 30 Minuten bei einem pH-Wert freisetzt, bei dem der Wirkstoff ausreichend löslich und stabil ist und bei dem eine entsprechende Arzneiform, die mit dem nicht neutralisierten anionischen (Meth)acrylat-Copolymer überzogen ist, weniger als 10 % des enthaltenen Wirkstoffs in 30 Minuten freisetzen würde.

Dem Fachmann ist der Freisetzungstest nach USP 28, insbesondere nach USP 28 <711> Paddle-Methode (= Apparatus 2), hinlänglich bekannt.

Die Wirkstofffreisetzung kann analog nach USP 28, insbesondere USP 28-NF23, General Chapter <711>, Dissolution, Apparatus 2 (Paddle), Method <724> "Delayed Release (Enteric Coated) Articles-General General Drug Release Standard", Method B (100 Upm, 37 °C) mit folgender Abwandlung bestimmt werden: Die überzogenen Arzneiformen werden in Analogie zu USP 28 beim jeweils relevanten pH-Wert in Pufferlösungen auf Wirkstofffreisetzung geprüft. Die Wirkstoffkonzentration im Testmedium kann abhängig vom Wirkstoff z. B. photometrisch oder mittels HPLC (High Pressure Liquid Chromatography) bestimmt werden.

### Mischungen mit weiteren filmbildenden Polymeren

Der Überzug der Arzneiform kann zusätzlich zu dem eines teilneutralisierten, anionischen (Meth)acrylat-Copolymeren bis zu 30, insbesondere bis zu 20 Gew.-% weiterer filmbildender Polymere, die keine oder nur in unwesentliche Mengen ionische Seitengruppen aufweisen, enthalten. Unwesentliche Mengen ionischer Seitengruppen sind im Polymeren vorhanden, wenn insgesamt weniger als 5 %, z. B. 0,1 bis weniger als 5 %, der Seitengruppen bzw. der Monomereinheiten, ionische Gruppen aufweisen.

Weitere filmbildende Polymere, die keine oder nur in unwesentliche Mengen ionische Seitengruppen aufweisen, können sein: Copolymere aus Methylmethacrylat und Ethylacrylat (EUDRAGIT^{®} NE30D), Polyvinylalkohol-Polyethylenglycol-Graft-Copolymer (Kollicoat® IR), Polyvinylacetat (PVAc, Kollicoat® SR), Hydroxyethylcellulose (HEC, Klucel®), Hydroxypropylcellulose (HPC), Hydroxypropylmethylcellulose (HPMC, Pharmacoat®, Methocel®, Sepifilm®, Viscontran®, Opadry®), Hydroxymehylethylcellulose (HEMC), Ethylcellulose (EC, Ethocel^{®}, Aquacoat^{®}, Surelease^{®}), Methylcellulose (MC, Viscontran®, Tylopur®, Methocel®), Celluloseester oder eine Mischung der genannten Polymere.

### Hilfsstoffe

Der verwendungsgemäßen Formulierung werden bevorzugt bei ihrer Herstellung übliche Zuschlagstoffe für den Aufbau des Kerns und für den Überzug hinzugefügt. Grundsätzlich müssen natürlich alle eingesetzten Substanzen toxikologisch unbedenklich und insbesondere in Arzneimitteln ohne Risiko für Patienten zu verwenden sein.

Einsatzmengen und Verwendung der üblichen Zuschlagstoffe in Arzneimittelüberzügen oder Beschichtungen sind dem Fachmann geläufig. Übliche Zuschlagstoffe können z. B. Trennmittel, Pigmente, Stabilisatoren, Antioxidantien, Porenbildner, Penetrationsförderer, Glanzmittel, Aromastoffe oder Geschmacksmittel sein. Sie dienen als Verarbeitungshilfsmittel und sollen ein sicheres und reproduzierbares Herstellungsverfahren sowie gute Langzeitlagerstabilität gewährleisten oder sie erreichen in der Arzneiform zusätzliche vorteilhafte Eigenschaften.

### • Weichmacher

Als Weichmacher geeignete Stoffe haben in der Regel ein Molekulargewicht zwischen 100 und 20 000 und enthalten eine oder mehrere hydrophile Gruppen im Molekül, z. B. Hydroxyl-, Ester- oder Aminogruppen. Geeignet sind Citrate, Phthalate, Sebacate, Rizinusöl. Beispiele geeigneter Weichmacher sind Citronensäurealkylester, Propylenglykol, Glycerinester, Phthalsäurealkylester, Sebacinsäurealkylester, Sucroseester, Sorbitanester, Diethylsebacat , Dibutylsebacat und Polyethylenglykole 4000 bis 20.000. Bevorzugte Weichmacher sind Tributylcitrat, Triethylcitrat, Acetyltriethylcitrat, Dibutylsebacat und Diethylsebacat. Die Einsatzmengen liegen zwischen 1 und 35, bevorzugt 5 bis 25 Gew.-% .-%, bezogen auf das teilneutralisierte (Meth)acrylat-Copolymere.

Die Zugabe der Weichmacher zur Formulierung kann in bekannter Weise, direkt, in wäßriger Lösung oder nach thermischer Vorbehandlung der Mischung vorgenommen werden. Auch können Mischungen von Weichmachern eingesetzt werden

### • Trennmittel:

Trennmittel besitzen in der Regel lipophile Eigenschaften und werden in der Regel den Sprühsuspensionen zugesetzt. Sie verhindern eine Agglomeration der Kerne während der Befilmung. Bevorzugt werden Talkum, Mg- oder Ca - Stearat, gemahlene Kieselsäure, Kaolin oder nicht ionische Emulgatoren mit einem HLB - Wert zwischen 3 und 8 eingesetzt. Übliche Einsatzmengen für Trennmittel liegen zwischen 0,5 bis 100 Gew.-% bezogen auf das Polymer oder die Polymermischung.

### • Pigmente:

Pigmente dienen der Färbung des Überzugs.

Pigmente können der (Meth)acrylat-Copolymer-Dispersion direkt zugesetzt werden, z. B. durch Einrühren, oder auch separat dispergiert werden und dann der Dispersion zugesetzt werden. Übliche Anwendungsmengen sind z. B. 20 bis 400 Gew.-%, bezogen auf das Gewicht des Polymeren oder der Polymermischung.

Siehe dazu z. B. auch: Deutsche Forschungsgemeinschaft, Farbstoffe für Lebensmittel, Harald Boldt Verlag KG, Boppard (1978); Deutsche Lebensmittelrundschau 74, Nr. 4, S. 156 (1978); Arzneimittelfarbstoffverordnung AmFarbV vom 25.08.1980.

Geeignete Pigmente können z. B. Aluminiumoxidpigmente oder Eisenoxidpigmente sein. Geeignet sind z. B. Gelborange , Cochenillerotlack, Farbpigmente auf Basis von Aluminiumoxid bzw Azofarbstoffen, Sulfonsäurefarbstoffe, Gelborange S (E110, C.I. 15985, FD&C Yellow 6), Indigocarmin (E132, C.I. 73015, FD&C Blue 2), Tartrazin (E 102, C.I. 19140, FD&C Yellow 5), Ponceau 4R (E 125, C.I. 16255, FD&C Cochineal Red A), Chinolingleb (E 104, C.I. 47005, FD&C Yellow 10), Erythrosin (E127, C.I. 45430, FD&C Red 3), Azorubin (E 122, C.I. 14720, FD&C Carmoisine), Amaranth (E 123, C.I. 16185, FD&C Red 2), Brilliantsäuregrün (E 142, C.I. 44090, FD&C Green S).

Die angegebenen E-Nummern der Pigmente beziehen sich auf eine EU-Nummerierung. Siehe dazu auch "Deutsche Forschungsgemeinschaft, Farbstoffe für Lebensmittel, Harald Boldt Verlag KG, Boppard (1978); Deutsche Lebensmittelrundschau 74, Nr. 4, S. 156 (1978); Arzneimittelfarbstoffverordnung AmFarbV vom 25.08.1980. Die FD&C-Nummern beziehen sich auf die Zulassung in Food, Drugs und Cosmetics durch U.S. Food and Drug Administration (FDA) beschrieben in: U.S. Food and Drug Administration, Center for Food Safety and Applied Nutrition, Office of Cosmetics and Colors: Code of Federal Regulations - Title 21 Color Additive Regulations Part 82, Listing of Certified Provisionally Listed Colors and Specifications (CFR 21 Part 82).

### Verwendungsgemäße Arzneiform

Die Erfindung betrifft die Verwendung eines teilneutralisierten anionischen (Meth)acrylat-Copolymers, bestehend aus radikalisch polymerisierten Einheiten von 40 bis 60 Gew.-% C₁- bis C₄-Alkylestern der Acryl- oder der Methacrylsäure und 40 bis 60 Gew.-% (Meth)acrylat-Monomeren mit einer anionischen Gruppe, wobei mehr als 10 bis 50 %, bevorzugt mehr als 10 bis 20 % oder bevorzugt mehr als 20 bis 40 % der enthaltenen anionischen Gruppen mittels einer Base neutralisiert sind, zur Herstellung einer Arzneiform mit wirkstoffhaltigem Kern, die mit dem teilneutralisierten anionischen (Meth)acrylat-Copolymer überzogen ist und mindestens 30 % des enthaltenen Wirkstoffs in 30 Minuten bei einem pH-Wert freisetzt, bei dem der Wirkstoff ausreichend löslich und stabil ist und bei dem eine entsprechende Arzneiform, die mit dem nicht neutralisierten anionischen (Meth)acrylat-Copolymer überzogen ist, weniger als 10 % des enthaltenen Wirkstoffs in 30 Minuten freisetzen würde, wobei der Überzug der Arzneiform bis zu 30 Gew.-% weiterer filmbildender Polymere, die keine oder nur in unwesentliche Mengen ionische Seitengruppen aufweisen, enthalten kann.

Die Arzneiform kann bevorzugt einen Polymerüberzug mit NaOH als Neutralisationsmittel in Kombination mit 5 bis 25 Gew.-% eines Weichmachers enthalten.

### Wirkstoffe

Verwendungsgemäß wird eine Arzneiform mit wirkstoffhaltigem Kern erhalten, die mit dem teilneutralisierten anionischen (Meth)acrylat-Copolymer überzogen ist und mindestens 30 % des enthaltenen Wirkstoffs in 30 Minuten bei einem pH-Wert freisetzt, bei dem der Wirkstoff ausreichend löslich und stabil ist. Eine verwendungsgemäße Arzneiform kann beispielsweise mit einem zu 15 % mit NaOH teilneutralisierten EUDRAGIT® L100-55 (Copolymer aus 50 Gew.-% Ethylacrylat und 50 Gew.-% Methacrylsäure; Spez. Auflösungs-pH-Wert = pH 5,5) überzogen sein (s. Bsp. C). In diesem Fall werden bereits bei pH 4,0 signifikante Mengen des enthaltenen Wirkstoffs freigesetzt. Der enthaltene Wirkstoff muss daher bei pH 4,0 ausreichend löslich und stabil sein, damit sich die beabsichtigte therapeutische Wirkung einstellen kann.

Unter ausreichend löslich wird gemäss dem Guidance for Industry "Waiver of In Vivo Bioavailability and Bioequivalence Studies for Immediate-Release Solid Oral Dosage Forms Based on a Biopharmaceutics Classification" by U.S. Department of Health and Human Services - Food and Drug Administration-Center for Drug Evaluation and Research CDER), August 2000, BP System" Abschnitt II.A - Solubility vertanden, dass sich eine Arzneistoffdosis unabhängig von ihrer absoluten Menge in 250 ml einer Pufferlösung mit dem jeweils relevanten pH-Wert löst. Der relevante pH-Werte wäre in obigem Beispiel pH 4,0. Die Bestimmung der Löslichkeit erfolgt bevorzugt analog der Vorschrift in dem Abschnitt III A.

Unter ausreichend stabil wird verstanden, wenn der enthaltene Wirkstoff einer Pufferlösung mit dem jeweils relevanten pH-Wert bei 37 °C über 2 Stunden zu einem Anteil von mindestens 90, bevorzugt von mindestens 95 %, therapeutisch wirksam oder chemisch unverändert bleibt, wobei ein gegebenenfalls zersetzter oder therapeutisch unwirksamer Anteil nicht toxisch oder unverträglich sein darf. Die pH-abhängige Stabilität kann durch chemischphysikalische Analyse, sowie durch in-vitro oder in-vivo Studien überprüft werden. Für eine Vielzahl gängiger Wirkstoffe liegen entsprechende Daten bereits vor. Acetyl-Salicylsäure ist z. B. bei pH 4,0 ausreichend löslich und stabil.

Geeignete Wirkstoffe sind für die Zwecke der Erfindung sind z. B. solche Wirkstoffe, deren Handelsformen magensaftresistente Überzüge aufweisen, weil die Wirkstoff bei pH 1,2 unlöslich oder instabil sind, von denen aber bekannt ist, dass sie ab etwa pH 3,0 ausreichend löslich und stabil sind. Der vorteilhafte Effekt der Erfindung tritt insbesondere bei solchen Wirkstoffen hervor, bei denen eine besonders rasche Wirkung indiziert ist.

Eine Auswahl solcher potentiell geeigneter Wirkstoffe können z. B. sein: Protonenpumpenblocker, insbesondere Omeprazol, Lanzoprazol, Pantoprazol, Rabeprazol, Perprazol, Esomprazol, Tenatoprazol, Anastrozol, Aripiprazol, Dapriprazol, Hydroxyomeprazol, Leminoprazol, sowie Paroxetin, Pindolol, Reboxetin, Antibiotika, insbesondere 5-Aminosalicylsäure, Sulfasalazin Budenosid, Natamycin, Preglumetacin Sulfasalacin, Nitrofurantion, Didanosin, nicht steroidale Antirheumatika, insbesondere Acetylsalicylsäure, Acemetacin, Ibuprofen, Diclofenac, Naproxen, Ketoprofen, Dexketoprofen, Indometacin, Tiaprofensäure, sowie Lipidsenker, insbesondere Pravastatin, Antikonvulsiva, insbesondere Valproinsäure oder Antihypertonica, wie z.B. Ramipril sowie deren Salze.

Weiterhin zu nennen sind Enzym-, Protein- oder Peptid-Wirkstoffe wie z.B.: Bromelain, Pankreatin oder Trypsin, ein Insulin, ein Human Growth Hormon (hGH), Corbaplatin, Intron A, Calcitonin, Cromalyn, ein Interferon, ein Calcitonin, Granulocyte Colony Stimulating factor (G-CSF), ein Interleukin, ein Kinin, Parathyroidhormone, Glucagon, Pro-Somatostatin, ein Somatostatin, Detirelix, Cetrorelix, Vasopressin, 1-Deaminocysteine-8-D-arginine-Vasopressin, Leuprolidacetat oder ein Antigen, das aus Gräsern oder anderen Pflanzen, wie z. B. Roggen, Weizen, Gerste, Hafer, Bermuda Gras, Zinnkraut, Ahorn, Ulme, Eiche, Platane, Pappel, Zeder, Zinnkraut, Disteln gewonnen wurde, IgG, spezifische Impfstoffe oder monoclonale Antikörper, ein Peptidhormon, ein immunmodulatorisches Protein, ein Antigen oder Antikörper.

Weitere Wirkstoffe, bei denen die Erfindung vorteilhaft eingesetzt werden kann sind: Oligonucleotide oder anionische Wirkstoffe mit einem pKₐ-Wert in fraglichen Bereich.

### Verfahren zur Herstellung einer verwendungsgemäßen Arzneiform

Die verwendungsgemäße Arzneiform kann in an sich bekannter Weise mittels pharmazeutisch üblicher Verfahren, wie direktem Verpressen, Verpressen von Trocken-, Feucht- oder Sintergranulaten, Extrusion und anschließende Ausrundung, feuchte oder trockene Granulation oder direkte Pelletierung oder durch Binden von Pulvern (Powder layering) auf wirkstofffreie Kugeln bzw. neutrale Kerne (Nonpareilles) oder wirkstoffhaltige Partikeln und mittels Auftrag des Polymerüberzugs im Sprühverfahren oder durch Wirbelschichtgranulation.

Die Schichtdicke des Überzugs ist über weite Bereiche ohne wesentlichen Einfluss auf den Freigabe pH-Wert. Übliche Auftragsmengen liegen zwischen 4 und 10 mg/Polymer pro cm² Kernoberfläche.

### Herstellung multipartikulärer Arzneiformen

Die Erfindung eignet sich auch zur Herstellung multipartikulärer Arzneiformen, da die zu verwendenden Copolymere den hohen Drücken beim Verpressen der Pellets mit dem Füllstoff standhalten.

Die Herstellung von multipartikulären Arzneiformen durch Verpressen eines pharmazeutisch üblichen Bindemittels mit wirkstoffhaltigen Partikeln ist z. B. Beckert et al. (1996), "Compression of enteric-coated pellets to disintegrating tablets", International Journal of Pharmaceutics 143, S. 13 - 23, und in WO 96/01624 ausführlich beschrieben.

Wirkstoffhaltige Pellets können hergestellt werden indem man mittels eines Layeringprozesses Wirkstoff aufbringt. Dazu wird Wirkstoff gemeinsam mit weiteren Hilfsstoffen (Trennmittel, ggf. Weichmacher) homogenisiert und in einem Bindemittel gelöst oder suspendiert. Mittels eines Wirbelschichtverfahrens kann die Flüssigkeit auf Placebopellets oder sonstige geeignete Trägermaterialien aufgebracht werden, wobei das Lösungs- oder Suspendiermittel verdunstet wird (Literatur: International Journal of Pharmaceutics 143, S. 13 - 23*).* Nach dem Herstellverfahren kann sich ein Trocknungsschritt anschließen. Der Wirkstoff kann in mehreren Schichten aufgebracht werden.

Einige Wirkstoffe, z. B. Acetylsalicylsäure, sind in Form von Wirkstoffkristallen handelsüblich und können in dieser Form anstelle von wirkstoffhaltigen Pellets eingesetzt werden.

Filmüberzüge auf wirkstoffhaltige Pellets werden üblicherweise in Wirbelschichtgeräten aufgebracht. Rezepturbeispiele sind in dieser Anmeldung erwähnt. Filmbildner werden üblicherweise mit Weichmachern und Trennmitteln nach einem geeigneten Verfahren gemischt. Hierbei können die Filmbildner als Lösung oder Suspension vorliegen. Die Hilfsstoffe für die Filmbildung können ebenfalls gelöst oder suspendiert sein. Organische oder wässrige Löse- oder Dispergiermittel können verwendet werden. Zur Stabilisierung der Dispersion können zusätzlich Stabilisatoren verwendet werden (Beispiel: Polysorbat 80 oder andere geeignete Emulgatoren bzw. Stabilisatoren).

Beispiele für Trennmittel sind Glycerolmonostearat oder andere geeignete Fettsäurederivate, Kieselsäurederivate oder Talkum. Beispiele für Weichmacher sind Propylenglykol, Phthalate, Polyethylenglykole, Sebacate oder Citrate, sowie andere in der Literatur erwähnte Substanzen.

Zwischen wirkstoffhaltiger und darmlöslicher Copolymer-Schicht kann eine trennende Schicht aufgebracht sein, die der Trennung von Wirkstoff und Überzugsmaterial zum Zwecke der Verhinderung von Interaktionen dient. Diese Schicht kann aus inerten Filmbildnern (z.B. HPMC, HPC oder (Meth)acrylsäure-Copolymeren) oder z.B. Talkum oder anderen geeigneten pharmazeutischen Substanzen bestehen. Ebenso können Kombinationen aus Filmbildnern und Talkum oder ähnlichen Stoffen verwendet werden.

Mischungen zur Herstellung von Tabletten aus überzogenen Partikeln werden durch Vermischen der Pellets mit geeigneten Bindemitteln für die Tablettierung, nötigenfalls der Zugabe von zerfallsfördernden Substanzen und nötigenfalls der Zugabe von Schmiermitteln zubereitet. Das Mischen kann in geeigneten Maschinen stattfinden. Ungeeignet sind Mischer, die zu Schäden an den überzogenen Partikeln führen, z. B. Pflugscharmischer. Zur Erzielung geeigneter kurzer Zerfallszeiten kann eine spezielle Reihenfolge bei der Zugabe der Hilfsstoffe zu den überzogenen Partikeln erforderlich sein. Durch Vormischung mit der überzogenen Partikel mit dem Schmier- oder Formentrennmittel Magnesiumstearat kann dessen Oberfläche hydrophobisiert und somit Verkleben vermieden werden.

Zum Tablettieren geeignete Mischungen enthalten üblicherweise 3 bis 15 Gew.-% eines Zerfallshilfsmittels, z. B. Kollidon CL und z. B. 0,1 bis 1 Gew.-% eines Schmier- und Formentrennmittels wie Magnesiumstearat. Der Bindemittelanteil bestimmt sich nach dem geforderten Anteil an überzogenen Partikeln. Typische Bindemittel sind z. B. Cellactose^{®}, mikrokristalline Cellulose, Calciumphosphate, Ludipress^{®}, Lactose oder andere geeignete Zucker, Calciumsulfate oder Stärkederivate. Bevorzugt werden Substanzen mit geringer Schüttdichte.

Typische Zerfallshilfsmittel (Sprengmittel) sind quervernetzte Stärke- oder Cellulosederivate, sowie quervernetztes Polyvinylpyrrolidon. Ebenso sind Cellulosederivate geeignet. Durch Auswahl eines geeigneten Bindemittels kann die Verwendung von Zerfallshilfsmittel entfallen.

Typische Schmier- und Formentrennmittel sind Magnesiumstearate oder andere geeignete Salze von Fettsäuren oder in der Literatur zu diesem Zweck aufgeführte Substanzen (z.B. Laurinsäure, Calciumstearat, Talkum usw.). Bei Verwendung geeigneter Maschinen (z.B. Tablettenpresse mit externer Schmierung) oder geeigneter Formulierungen kann die Verwendung eines Schmier- und Formentrennmittels in der Mischung entfallen.

Der Mischung kann gegebenenfalls ein Hilfsmittel zur Fließverbesserung beigefügt sein (z. B. hochdisperse Kieselsäurederivate, Talkum usw.).

Das Tablettieren kann auf üblichen Tablettenpressen, Exzenter- oder Rundlauftablettenpressen erfolgen, bei Preßkräften im Bereich von 5 bis 40 kN, bevorzugt 10 - 20 kN. Die Tablettenpressen können mit Systemen zur externen Schmierung ausgestattet sein. Gegebenenfalls kommen spezielle Systeme zur Matrizenbefüllung zum Einsatz, die die Matrizenbefüllung mittels Rührflügeln vermeiden.

Die Schichtdicke des Überzugs ist über weite Bereiche ohne wesentlichen Einfluss auf den Freigabe pH-Wert. Übliche Auftragsmengen liegen zwischen 4 und 10 mg/Polymer pro cm² Kernoberfläche.

### Weitere Herstellungsverfahren für die verwendungsgemässe Arzneiform

Auftragsverfahren erfolgt mittels Sprühauftrag aus organischer Lösung, oder bevorzugt mittels Sprühauftrag aus wäßrigen Dispersionen, durch Schmelzen oder durch direkten Pulverauftrag. Für die Ausführung ist dabei entscheidend, daß gleichmäßige, porenfreie Überzüge entstehen.

Auftragsverfahren gemäß Stand der Technik s. z.B. Bauer, Lehmann, Osterwald, Rothgang, "Überzogene Arzneiformen" Wissenschaftliche Verlagsgesellschaft mbH Stuttgart, Kap 7, S.165 - 196

Für die Applikation sind relevante Eigenschaften, geforderte Tests und Spezifikationen in Arzneibüchern aufgelistet.

Details sind den gängigen Lehrbüchern zu entnehmen, z.B.:
- Voigt, R. (1984): Lehrbuch der pharmazeutischen Technologie; Verlag Chemie Weinheim - Beerfield Beach/Florida - Basel.
- Sucker, H., Fuchs, P., Speiser, P. : Pharmazeutische Technologie, Georg Thieme Verlag Stuttgart (1991), insbesondere Kapitel 15 und 16, S. 626 - 642.
- Gennaro, A.,R. (Editor), Remington's Pharmaceutical Sciences, Mack Publishing Co., Easton Pennsylvania (1985), Chapter 88, S. 1567 - 1573.
- List, P. H. (1982): Arzneiformenlehre, Wissenschaftliche Verlagsgesellschaft mbH, Stuttgart.

### Deckschicht

Es ist auch möglich eine äußere Deckschicht (Topcoat) aus einem weiteren, bevorzugt wasserlöslichen, Polymeren und Hilfsstoffen, z. B. Pigmenten und/oder Trennmitteln, aufzubringen, die weitere Funktionen gewährleistet, wie beispielsweise Färbung oder Verhinderung von Verkleben.

### Trennschicht

Zwischen wirkstoffhaltiger und darmlöslicher Copolymer-Schicht kann eine trennende Schicht (Subcoat) aufgebracht sein, die der Trennung von Wirkstoff und Überzugsmaterial zum Zwecke der Verhinderung von Interaktionen dient. Diese Schicht kann aus inerten Filmbildnern (z.B. HPMC, HPC oder (Meth)acrylsäure-Copolymeren) oder z.B. Talkum oder anderen geeigneten pharmazeutischen Substanzen bestehen. Ebenso können Kombinationen aus Filmbildnern und Talkum oder ähnlichen Stoffen verwendet werden.

### BEISPIELE

Überzug mit EUDRAGIT® L100-55 (Copolymer aus 50 Gew.-% Ethylacrylat und 50 Gew.-% Methacrylsäure) aus 30%-iger Dispersion (EUDRAGIT® L30D-55). Spezifischer Auflösungs-pH-Wert = pH 5,5. Teilneutralisation mit NaOH.

In den Beispielen A bis D wurden Theophyllingranulate, Teilchengröße 0,5 bis 0,8 mm, bei Produkttemperaturen zwischen 30 und 35 °C, mit nicht neutralisiertem und mit unterschiedlich teilneutralisierten Polymeren in einem Wirbelschichtgerät überzogen. Der Polymerauftrag lag in allen Versuchen zwischen 18 und 25 Gew.-%, bezogen auf das Kerngewicht

| Rezeptur (Beispiel A): | | | |
|---|---|---|---|
| | Menge | Feststoff | % Feststoff |
| EUDRAGIT L 30 D - 55 | 794,1 g | 238,2 g | 30,0 |
| Talkum | 119,1 g | 119,1 g | 50,0 |
| Triethylcitrat | 23,8 g | 23,8 g | 10,0 |
| Wasser | 1456,7 g | | |

In den Beispielen B, C und D wurde im Unterschied zu Beispiel A 1 N NaOH in entsprechende Menge, um 4,4, 15 oder 30 % der Carboxylgruppen von EUDRAGIT L 30 D - 55 zu neutralisieren, zugegeben.
Nach dem Sprühauftrag wurden die Partikel für 2 Stunden auf Horden bei 40 °C getrocknet.

### Freisetzungstest von Theophyllin-Pellets nach USP 28 <711> Paddle-Methode (= Apparatus 2)

Die überzogenen Theophyllin-Partikel wurden nach USP 28 <711> Paddle-Methode (= Apparatus 2) bei 100 Upm getestet.

Anschließend wurde die Wirkstofffreigabe analog USP 28 mit folgenden Pufferkompositionen (Tabelle 1) bei konstantem pH-Wert geprüft. Die Wirkstofffreisetzung nach 30 min wurde photometrisch bestimmt (s. Tabelle 2).

**Tabelle 1**

| | pH 2 | pH 3 | pH 4 | pH 5 | pH 6 |
|---|---|---|---|---|---|
| | | | | | |
| H₃PO₄ 0.1 M | 2210 g | 680 g | 553 g | 509 g | 332 g |
| NaCI | 42.5 g | 42.5 g | 42.5 g | 42.5 g | 42.5 g |
| KH₂PO₄ | 5 g | 5 g | 5 g | 5 g | 5 g |
| K₂HPO₄ | 10g | 10 g | 10 g | 10 g | 10 g |
| Water ad | 5000 ml | 5000 ml | 5000 ml | 5000 ml | 5000 ml |

Beispiele A und B: nicht erfindungsgemäß. Beispiele A und B dienen als Vergleich für die erfindungsgemäßen Beispiele C und D.

EUDRAGIT® L30D-55: Spezifischer Auflösungs-pH-Wert = pH 5,5

**Tabelle 2**

| Beispiel | A | B | C | D |
|---|---|---|---|---|
| | Vergleich | Vergleich | erfindungsgemäß | erfindungsgemäß |
| | EUDRAGIT® L100-55 (Copolymer aus 50 Gew.-% Ethylacrylat und 50 Gew.-% Methacrylsäure): Spez. Auflösungs-pH-Wert = pH 5,5 | | | |

| Neutralisationsgrad [%] | 0 | 4,4 | 15 | 30 |
|---|---|---|---|---|
| pH-Wert | Wirkstofffreisetzung [%] nach 30 Minuten | | | |
| 2,0 | 0 | 0 | 0 | 6 |
| 3,0 | 0 | 0 | 5 | 35 |
| 4,0 | 0 | 3 | 35 | 95 |
| 5,0 | 0 | 33 | 70 | 99 |
| 6,0 | 98 | 100 | 98 | 97 |

## Patentansprüche

1. Verwendung eines teilneutralisierten, anionischen (Meth)acrylat-Copolymers, bestehend aus radikalisch polymerisierten Einheiten von 40 bis 60 Gew.-% C₁-bis C₄-Alkylestern der Acryl- oder der Methacrylsäure und 60 bis 40 Gew.-% (Meth)acrylat-Monomeren mit einer anionischen Gruppe, wobei mehr als 10 bis 50 % der enthaltenen anionischen Gruppen mittels einer Base neutralisiert sind,
zur Herstellung einer Arzneiform mit wirkstoffhaltigem Kern, die mit dem teilneutralisierten, anionischen (Meth)acrylat-Copolymer überzogen ist und mindestens 30 % des enthaltenen Wirkstoffs in 30 Minuten bei einem pH-Wert freisetzt, bei dem der Wirkstoff ausreichend löslich und stabil ist und bei dem eine entsprechende Arzneiform, die mit dem nicht neutralisierten anionischen (Meth)acrylat-Copolymer überzogen ist, weniger als 10 % des enthaltenen Wirkstoffs in 30 Minuten freisetzen würde,
wobei der Überzug der Arzneiform bis zu 30 Gew.-% weiterer filmbildender Polymere, die keine oder nur in unwesentliche Mengen ionische Seitengruppen aufweisen, enthalten kann.

2. Verwendung nach Anspruch 1, **dadurch gekennzeichnet, dass** die Base, die zur Teilneutralisation eingesetzt wird, ein Molekulargewicht von höchstens 150 aufweist.

3. Verwendung nach Anspruch 1 oder 2, **dadurch gekennzeichnet, dass** NaOH, KOH oder Ammoniak zur Teilneutralisation eingesetzt wird.

4. Verwendung nach Anspruch 1 oder 3, **dadurch gekennzeichnet, dass** der Teilneutralisationsgrad mehr als 10 bis 20 % beträgt.

5. Verwendung nach Anspruch 4, **dadurch gekennzeichnet, dass** der pH-Wert, bei dem mindestens 30 % des enthaltenen Wirkstoffs in 30 Minuten freigesetzt werden und bei dem der Wirkstoff ausreichend löslich und stabil ist und bei dem eine entsprechende Arzneiform, die mit dem nicht neutralisierten anionischen (Meth)acrylat-Copolymer überzogen ist, weniger als 10 % des enthaltenen Wirkstoffs in 30 Minuten freisetzen würde, um 1,5 bis 2,5 pH-Einheiten unter dem pH-Wert des Beginns der spezifischen Wirkstofffreisetzung in Darmsaft oder künstlichem Darmsaft eines nicht teilneutralisierten, anionischen (Meth)acrylat-Copolymers liegt.

6. Verwendung nach Anspruch 1 oder 3, **dadurch gekennzeichnet, dass** der Teilneutralisationsgrad mehr als 20 bis 40 % beträgt.

7. Verwendung nach Anspruch 6, **dadurch gekennzeichnet, dass** der pH-Wert, bei dem mindestens 30 % des enthaltenen Wirkstoffs in 30 Minuten freigesetzt werden und bei dem der Wirkstoff ausreichend löslich und stabil ist und bei dem eine entsprechende Arzneiform, die mit dem nicht neutralisierten anionischen (Meth)acrylat-Copolymer überzogen ist, weniger als 10 % des enthaltenen Wirkstoffs in 30 Minuten freisetzen würde, um 2,5 bis 3,5 pH-Einheiten unter dem pH-Wert des Beginns der spezifischen Wirkstofffreisetzung in Darmsaft oder künstlichem Darmsaft eines nicht teilneutralisierten, anionischen (Meth)acrylat-Copolymers liegt.

8. Verwendung nach einem oder mehreren der Ansprüche 1 bis 7, **dadurch gekennzeichnet, dass** das anionische (Meth)acrylat-Copolymer aus radikalisch polymerisierten Einheiten von 40 bis 60, Gew.-% Methacrylsäure und 60 bis 40 Gew.-% Methylmethacrylat oder 60 bis 40 Gew.-% Ethylacrylat besteht.

9. Verwendung nach einem oder mehreren der Ansprüche 1 bis 8, **dadurch gekennzeichnet, dass** das anionische (Meth)acrylat-Copolymer in Form einer wäßrigen Dispersion mit 10 bis 50-prozentigen Feststoffanteil eingesetzt wird.

10. Verwendung nach Anspruch 1, **dadurch gekennzeichnet, dass** als weitere filmbildende Polymere, die keine oder nur in unwesentliche Mengen ionische Seitengruppen aufweisen, ein Copolymer aus Methylmethacrylat und Ethylacrylat (EUDRAGIT^{®} NE30D), Polyvinylalkohol-Polyethylenglycol-Graft-Copolymer (Kollicoat® IR), Polyvinylacetat (PVAc, Kollicoat® SR), Hydroxyethylcellulose (HEC, Klucel®), Hydroxypropylcellulose (HPC), Hydroxypropylmethylcellulose (HPMC, Pharmacoat®, Methocel®, Sepifilm®, Viscontran®, Opadry®), Hydroxymehylethylcellulose (HEMC), Ethylcellulose (EC, Ethocel^{®}, Aquacoat^{®}, Surelease^{®}), Methylcellulose (MC, Viscontran®, Tylopur®, Methocel®), Celluloseester oder eine Mischung der genannten Polymere eingesetzt wird.

11. Verwendung nach einem oder mehreren der Ansprüche 1 bis 10, **dadurch gekennzeichnet, dass** Protonenpumpenblocker, insbesondere Omeprazol, Lanzoprazol, Pantoprazol, Rabeprazol, Perprazol, Esomprazol, Tenatoprazol, Anastrozol, Aripiprazol, Dapriprazol, Hydroxyomeprazol, Leminoprazol, sowie Paroxetin, Pindolol, Reboxetin, Antibiotika, insbesondere 5-Aminosalicylsäure, Sulfasalazin Budenosid, Natamycin, Preglumetacin Sulfasalacin, Nitrofurantion, Didanosin, nicht steroidale Antirheumatika, insbesondere Acetylsalicylsäure, Acemetacin, Ibuprofen, Diclofenac, Naproxen, Ketoprofen, Dexketoprofen, Indometacin, Tiaprofensäure, sowie Lipidsenker, insbesondere Pravastatin, Antikonvulsiva, insbesondere Valproinsäure oder Antihypertonica, wie z.B. Ramipril sowie deren Salze als Wirkstoffe enthalten sind.

12. Verwendung nach einem oder mehreren der Ansprüche 1 bis 11, **dadurch gekennzeichnet, dass** als Wirkstoff ein Protonenpumpenblocker und als Überzug ein teilneutralisiertes, anionisches (Meth)acrylat-Copolymer aus radikalisch polymerisierten Einheiten von 40 bis 60 Gew.-% Methacrylsäure und 60 bis 40 Gew.-% Ethylacrylat eingesetzt wird.

13. Verwendung nach einem oder mehreren der Ansprüche 1 bis 3 oder 6 bis 11, **dadurch gekennzeichnet, dass** als Wirkstoff Acetylsalicylsäure und als Überzug ein zu mehr als 20 bis 40 % teilneutralisiertes, anionisches (Meth)acrylat-Copolymer aus radikalisch polymerisierten Einheiten von 40 bis 60 Gew.-% Methacrylsäure und 60 bis 40 Gew.-% Ethylacrylat eingesetzt wird.

## Claims

1. Use of a partially neutralized, anionic (meth)acrylate copolymer, consisting of free radical-polymerized units of 40 to 60% by weight of C₁- to C₄-alkyl esters of acrylic or of methacrylic acid and 60 to 40% by weight of (meth)acrylate monomers having an anionic group, more than 10 to 50% of the anionic groups contained being neutralized by means of a base,
for the production of a pharmaceutical form having an active substance-containing core, which is coated with the partially neutralized, anionic (meth)acrylate copolymer and releases at least 30% of the active substance contained in 30 minutes at a pH at which the active substance is adequately soluble and stable and at which a corresponding pharmaceutical form which is coated with the un-neutralized anionic (meth)acrylate copolymer would release less than 10% of the active substance contained in 30 minutes,
the coating of the pharmaceutical form being able to contain up to 30% by weight of further film-forming polymers which contain no or only insignificant amounts of ionic side groups.

2. Use according to Claim 1, **characterized in that** the base which is employed for the partial neutralization has a molecular weight of at most 150.

3. Use according to Claim 1 or 2, **characterized in that** NaOH, KOH or ammonia is employed for the partial neutralization.

4. Use according to Claim 1 or 3, **characterized in that** the degree of partial neutralization is more than 10 to 20%.

5. Use according to Claim 4, **characterized in that** the pH at which at least 30% of the active substance contained is released in 30 minutes and at which the active substance is adequately soluble and stable and at which a corresponding pharmaceutical form which is coated with the un-neutralized anionic (meth)acrylate copolymer would release less than 10% of the active substance contained in 30 minutes lies around 1.5 to 2.5 pH units below the pH of the start of the specific active substance release in intestinal juice or artificial intestinal juice of a not partially neutralized, anionic (meth)acrylate copolymer.

6. Use according to Claim 1 or 3, **characterized in that** the degree of partial neutralization is more than 20 to 40%.

7. Use according to Claim 6, **characterized in that** the pH at which at least 30% of the active substance contained is released in 30 minutes and at which the active substance is adequately soluble and stable and at which a corresponding pharmaceutical form which is coated with the un-neutralized anionic (meth)acrylate copolymer would release less than 10% of the active substance contained in 30 minutes lies around 2.5 to 3.5 pH units below the pH of the start of the specific active substance release in intestinal juice or artificial intestinal juice of a not partially neutralized, anionic (meth)acrylate copolymer.

8. Use according to one or more of Claims 1 to 7, **characterized in that** the anionic (meth)acrylate copolymer consists of free radical-polymerized units of 40 to 60% by weight of methacrylic acid and 60 to 40% by weight of methyl methacrylate or 60 to 40% by weight of ethyl acrylate.

9. Use according to one or more of Claims 1 to 8, **characterized in that** the anionic (meth)acrylate copolymer is employed in the form of an aqueous dispersion having a 10 to 50% solids content.

10. Use according to Claim 1, **characterized in that** a copolymer of methyl methacrylate and ethyl acrylate (EUDRAGIT^{®} NE30D), polyvinyl alcoholpolyethylene glycol graft copolymer (Kollicoat® IR), polyvinyl acetate (PVAc, Kollicoat® SR), hydroxyethylcellulose (HEC, Klucel®), hydroxypropylcellulose (HPC), hydroxypropylmethylcellulose (HPMC, Pharmacoat®, Methocel®, Sepifilm®, Viscontran®, Opadry®), hydroxymethylethylcellulose (HEMC), ethylcellulose (EC, Ethocel®, Aquacoat®, Surelease®), methylcellulose (MC, Viscontran®, Tylopur®, Methocel®), cellulose esters or a mixture of the polymers mentioned is used as further film-forming polymers which contain no or only insignificant amounts of ionic side groups.

11. Use according to one or more of Claims 1 to 10, **characterized in that** proton pump blockers, in particular omeprazole, lanzoprazole, pantoprazole, rabeprazole, perprazole, esomprazole, tenatoprazole, anastrozole, aripiprazole, dapriprazole, hydroxyomeprazole, leminoprazole, and paroxetine, pindolol, reboxetine, antibiotics, in particular 5-aminosalicylic acid, sulfasalazine budenoside, natamycin, preglumetacin, sulfasalacine, nitrofurantion, didanosine, nonsteroidal antirheumatics, in particular acetylsalicylic acid, acemetacin, ibuprofen, diclofenac, naproxen, ketoprofen, dexketoprofen, indometacin, tiaprofenic acid, and lipid-lowering agents, in particular pravastatin, anticonvulsants, in particular valproic acid, or antihypertensives, such as, for example, ramipril and its salts are contained as active substances.

12. Use according to one or more of Claims 1 to 11, **characterized in that** the active substance employed is a proton pump blocker and the coating employed is a partially neutralized, anionic (meth)acrylate copolymer of free radical-polymerized units of 40 to 60% by weight of methacrylic acid and 60 to 40% by weight of ethyl acrylate.

13. Use according to one or more of Claims 1 to 3 or 6 to 11, **characterized in that** the active substance employed is acetyl salicylic acid and the coating employed is an anionic (meth)acrylate copolymer, which is partially neutralized to more than 20 to 40%, of free radical-polymerized units of 40 to 60% by weight of methacrylic acid and 60 to 40% by weight of ethyl acrylate.

## Revendications

1. Utilisation d'un copolymère de (méth)acrylate anionique partiellement neutralisé, constitué par des unités polymérisées par voie radicalaire de 40 à 60 % en poids d'esters alkyliques en C₁ à C₄ de l'acide acrylique ou méthacrylique et 60 à 40 % en poids de monomères de (méth)acrylate contenant un groupe anionique, plus de 10 à 50 % des groupes anioniques contenus étant neutralisés avec une base,
pour la fabrication d'une forme médicamenteuse comprenant un noyau contenant un agent actif, qui est revêtue avec le copolymère de (méth)acrylate anionique partiellement neutralisé et qui libère au moins 30 % de l'agent actif contenu en 30 minutes à un pH auquel l'agent actif est suffisamment soluble et stable, et auquel une forme médicamenteuse correspondante qui est revêtue avec le copolymère de (méth)acrylate anionique non neutralisé libérerait moins de 10 % de l'agent actif contenu en 30 minutes,
le revêtement de la forme médicamenteuse pouvant contenir jusqu'à 30 % en poids d'autres polymères filmogènes, qui ne comprennent pas de groupes latéraux ioniques ou uniquement en quantités insignifiantes.

2. Utilisation selon la revendication 1, **caractérisée en ce que** la base utilisée pour la neutralisation partielle présente un poids moléculaire d'au plus 150.

3. Utilisation selon la revendication 1 ou 2, **caractérisée en ce que** NaOH, KOH ou l'ammoniac est utilisé pour la neutralisation partielle.

4. Utilisation selon la revendication 1 ou 3, **caractérisée en ce que** le degré de neutralisation partielle est de plus de 10 à 20 %.

5. Utilisation selon la revendication 4, **caractérisée en ce que** le pH auquel au moins 30 % de l'agent actif contenu est libéré en 30 minutes et auquel l'agent actif est suffisamment soluble et stable, et auquel une forme médicamenteuse correspondante qui est revêtue avec le copolymère de (méth)acrylate anionique non neutralisé libérerait moins de 10 % de l'agent actif contenu en 30 minutes, est 1,5 à 2,5 unités pH en dessous du pH du début de la libération spécifique de l'agent actif dans le suc intestinal ou le suc intestinal synthétique d'un copolymère de (méth)acrylate anionique non partiellement neutralisé.

6. Utilisation selon la revendication 1 ou 3, **caractérisée en ce que** le degré de neutralisation partielle est de plus de 20 à 40 %.

7. Utilisation selon la revendication 6, **caractérisée en ce que** le pH auquel au moins 30 % de l'agent actif contenu est libéré en 30 minutes et auquel l'agent actif est suffisamment soluble et stable, et auquel une forme médicamenteuse correspondante qui est revêtue avec le copolymère de (méth)acrylate anionique non neutralisé libérerait moins de 10 % de l'agent actif contenu en 30 minutes, est 2,5 à 3,5 unités pH en dessous du pH du début de la libération spécifique de l'agent actif dans le suc intestinal ou le suc intestinal synthétique d'un copolymère de (méth)acrylate anionique non partiellement neutralisé.

8. Utilisation selon une ou plusieurs des revendications 1 à 7, **caractérisée en ce que** le copolymère de (méth)acrylate anionique est constitué d'unités polymérisées par voie radicalaire de 40 à 60 % en poids d'acide méthacrylique et 60 à 40 % en poids de méthacrylate de méthyle ou 60 à 40 % en poids d'acrylate d'éthyle.

9. Utilisation selon une ou plusieurs des revendications 1 à 8, **caractérisée en ce que** le copolymère de (méth)acrylate anionique est utilisé sous la forme d'une dispersion aqueuse ayant une teneur en solides de 10 à 50 pour cent.

10. Utilisation selon la revendication 1, **caractérisée en ce que** un copolymère de méthacrylate de méthyle et d'acrylate d'éthyle (EUDRAGIT® NE30D), un copolymère greffé d'alcool polyvinylique-polyéthylène glycol (Kollicoat® IR), l'acétate de polyvinyle (PVAc, Kollicoat® SR), l'hydroxyéthylcellulose (HEC, Klucel®), l'hydroxypropylcellulose (HPC), l'hydroxypropylméthylcellulose (HPMC, Pharmacoat®, Methocel®, Sepifilm®, Viscontran®, Opadry®), l'hydroxyméthyléthylcellulose (HEMC), l'éthylcellulose (EC, Ethocel®, Aquacoat®, Surelease®), la méthylcellulose (MC, Viscontran®, Tylopur®, Methocel®), un ester de cellulose ou un mélange des polymères mentionnés est utilisé en tant qu'autres polymères filmogènes qui ne contiennent pas de groupes latéraux ioniques ou uniquement en quantités insignifiantes.

11. Utilisation selon une ou plusieurs des revendications 1 à 10, **caractérisée en ce que** des bloquants de la pompe à protons, notamment l'oméprazole, le lanzoprazole, le pantoprazole, le rabéprazole, le perprazole, l'ésomprazole, le ténatoprazole, l'anastrozole, l'aripiprazole, le dapriprazole, l'hydroxyoméprazole, le léminoprazole, ainsi que la paroxétine, le pindolol, la réboxétine, des antibiotiques, notamment l'acide 5-aminosalicylique, le sulfasalazine budénoside, la natamycine, la préglumétacine sulfasalacine, la nitrofurantoïne, la didanosine, des agents antirhumatismaux non stéroïdiens, notamment l'acide acétylsalicylique, l'acémétacine, l'ibuprofène, le diclofénac, le naproxène, le kétoprofène, le dexkétoprofène, l'indométacine, l'acide tiaprofénique, ainsi que des agents de réduction des lipides, notamment la pravastatine, des anticonvulsifs, notamment l'acide valproïque, ou des antihypertoniques, tels que p. ex. le ramipril, ainsi que leurs sels sont contenus en tant qu'agents actifs.

12. Utilisation selon une ou plusieurs des revendications 1 à 11, **caractérisée en ce qu'**un bloquant de la pompe à protons est utilisé en tant qu'agent actif et un copolymère de (méth)acrylate anionique partiellement neutralisé constitué d'unités polymérisées par voie radicalaire de 40 à 60 % en poids d'acide méthacrylique et 60 à 40 % en poids d'acrylate d'éthyle est utilisé en tant que revêtement.

13. Utilisation selon une ou plusieurs des revendications 1 à 3 ou 6 à 11, **caractérisée en ce que** l'acide acétylsalicylique est utilisé en tant qu'agent actif et un copolymère de (méth)acrylate anionique partiellement neutralisé à hauteur de plus de 20 à 40 %, constitué d'unités polymérisées par voie radicalaire de 40 à 60 % en poids d'acide méthacrylique et 60 à 40 % en poids d'acrylate d'éthyle est utilisé en tant que revêtement.
